(19) Europäisches Patentamt / European Patent Office / Office européen des brevets

(11) **EP 4 537 866 A1**

(12) **EUROPEAN PATENT APPLICATION**

(43) Date of publication:
16.04.2025 Bulletin 2025/16

(21) Application number: 23202446.3

(22) Date of filing: 09.10.2023

(51) International Patent Classification (IPC):
*A61M 3/02* (2006.01)  *A61M 25/10* (2013.01)

(52) Cooperative Patent Classification (CPC):
**A61M 3/0295; A61M 3/0202; A61M 3/0258;
A61M 25/1018; A61M 25/10184;** A61M 2205/3334;
A61M 2205/3584; A61M 2205/502; A61M 2205/52

| | |
|---|---|
| (84) Designated Contracting States:<br>**AL AT BE BG CH CY CZ DE DK EE ES FI FR GB<br>GR HR HU IE IS IT LI LT LU LV MC ME MK MT NL<br>NO PL PT RO RS SE SI SK SM TR**<br>Designated Extension States:<br>**BA**<br>Designated Validation States:<br>**KH MA MD TN** | (71) Applicant: **Wellspect AB<br>431 21 Mölndal (SE)**<br><br>(72) Inventor: **D Espindula, Gabriel<br>418 71 Göteborg (SE)**<br><br>(74) Representative: **Aldridge, Henry Alexander<br>Venner Shipley LLP<br>200 Aldersgate<br>London EC1A 4HD (GB)** |

(54) **AN IRRIGATION SYSTEM WITH IMPROVED INFLATION CONTROL**

(57)    An irrigation system for medical use comprises a catheter having an inflatable retention member, an electrically operated pump for pumping a fluid into the catheter for inflation of the inflatable retention member, and an electrically operated valve for deflation of the inflatable retention member. A controller is arrange to control the pump and the valve by determining an inflation flow rate into the inflatable retention member from the electrically operated pump and a deflation flow rate out from the inflatable retention member through the electrically operated valve, to continuously determine an inflation fluid volume based on the inflation flow rate, and a deflation fluid volume based on the deflation flow rate, to continuously determine a present fluid volume in the inflatable retention member as the inflation fluid volume reduced by the deflation fluid volume, and to prohibit operation of the electrically operated pump when the present fluid volume is determined to have reached a volume safety threshold.

Fig. 5

EP 4 537 866 A1

**Description**

TECHNICAL FIELD OF THE INVENTION

The present invention relates to an irrigation system comprising a catheter with an inflatable retention member. The irrigation system is particularly intended for rectal, transanal and/or stomal irrigation, and is suitable for self-administration of an irrigation liquid.

BACKGROUND OF THE INVENTION

Catheters and catheter systems are used for many types of medical procedures. In some catheter systems, the catheters are provided with retention members, to retain the catheters in place during use when inserted into the patient's/user's body. A common type of retention member for such catheters is an inflatable retention member, such as an inflatable balloon, arranged close to the insertable end of the catheter. A separate lumen is arranged within the catheter for transferring an inflation fluid from an external source, through the length of the catheter, and into the inflatable retention member for inflation, and for transferring the inflation fluid back though the length of the catheter during deflation of the inflatable retention member. The inflatable retention member is inflated to a suitable diameter with fluid, such as air, water or saline.

In addition to the lumen used for the inflation fluid, a second lumen may be provided to deliver e.g. irrigation fluid to the body cavity, for drainage of a liquid or the like, such as feces in case of a rectal catheter or urine in case of an indwelling urinary catheter, from the body cavity, etc.

The inflatable retention member preferably surrounds the insertion end of the catheter and preferably has a toroidal shape when fully inflated.

However, systems using an inflatable retention member must be used carefully because they can create too much pressure on the body tissue if the retention balloon is overinflated. Accordingly, all such catheter systems must have an indicated maximum volume for the retention balloon that each manufacturer has established as safe. However, this maximum volume can be exceeded by overinflating the inflatable retention member, resulting in potential damage to the soft tissue surrounding the retention member. In particular, overinflation to such a level that the inflatable retention member explodes may be both harmful and painful and may in severe cases even have lethal consequences. However, even without exploding, the inflated retention member can create discomfort, pain or even damage by creating too much pressure on the tissue in vicinity of the inflatable retention member.

Various attempts have been made over the years to find solutions to better control inflation of such inflatable retention members. However, most of these proposed solutions are very complex and costly to produce. Further, these known systems are often still not able to provide the user with adequate possibilities to control the inflation, and at the same time ensuring that overinflation is avoided.

For example, US 2016/0193403 by the same applicant discloses an irrigation system in which balloon inflation is controlled in accordance with pre-set inflation levels. However, the inflated size of the balloon may sometime vary from user to user, and also sometimes from time to time for the same user. The inflated size of the balloon may also vary depending on differences in the material of the balloon, due to product variations, tolerances, etc. Further, EP 2 683 424 discloses an electrically operated transanal irrigation system, where inflation of an inflatable balloon may be controlled by a pressure sensor. Still further, US 2014/0052063 discloses an irrigation system comprising a catheter with an inflatable balloon. A control system is provided for prevention of over-inflation of the balloon. To this end, the system monitors either the fluid pressure in the balloon as it is filled, or volume of fluid being provided to the balloon. Also, WO 2016/095929 discloses an electrically operated rectal irrigation system. It discloses the use of an electric pump for inflation of a balloon, and the pumping is controlled based on time duration or number of pump revolutions. EP 3 338 827 discloses a system and method for controlling inflation of an inflatable balloon where an electrically operated pump is controlled based on both a measured pressure in the balloon and a determined total duration of the pumping.

There is thus a need for improvements of the control of inflation of catheters having inflatable retention members in rectal or transanal irrigation systems, but also in other catheter systems having inflatable retention members, such as in urinary catheter systems, in endotracheal intubation systems etc. In particular, there is a need for an irrigation device which can be used safely, easily and conveniently, and with improved controllability, for self-administration of the irrigation liquid, and which also preferably can be produced in a cost-efficient way. It would be desirable to ensure the same or a similar performance in volume delivery regardless of degradations due to previous inflations, material variations, varying body pressures, etc.

SUMMARY OF THE INVENTION

It is therefore an object to provide an irrigation system for medical use which at least alleviates the above-discussed drawbacks of the prior art. In particular, it is an object to provide an irrigation system for use in irrigation procedures, such as in rectal or transanal irrigation.

This and other objects are achieved with an irrigation system according to the appended claims.

According to a first aspect, there is provided an irrigation system for medical use comprising:

a catheter having an inflatable retention member;
an electrically operated pump for pumping a fluid into the catheter for inflation of the inflatable retention member;
an electrically operated valve for deflation of the inflatable retention member; and
a controller for controlling the electrically operated pump and the electrically operated valve, the controller arranged to determine an inflation flow rate into the inflatable retention member from the electrically operated pump, and a deflation flow rate out from the inflatable retention member through the electrically operated valve, to continuously determine an inflation fluid volume based on the inflation flow rate and a deflation fluid volume based on the deflation flow rate, to continuously determine a present fluid volume in the inflatable retention member as the inflation fluid volume reduced by the deflation fluid volume, and to prohibit operation of the electrically operated pump when the present fluid volume is determined to have reached a volume safety threshold.

According to another aspect, there is provided an irrigation system for medical use comprising:

a catheter having an inflatable retention member;
an electrically operated reversible pump for pumping a fluid into the catheter for inflation of the inflatable retention member and out from the catheter for deflation of the inflatable retention member; and
a controller for controlling the electrically operated pump, the controller arranged to determine an inflation flow rate into the inflatable retention member from the electrically operated pump, and a deflation flow rate out from the inflatable retention member through the electrically operated pump, to continuously determine an inflation fluid volume based on the inflation flow rate, and a deflation fluid volume based on the deflation flow rate, to continuously determine a present fluid volume in the inflatable retention member as the inflation fluid volume reduced by the deflation fluid volume, and to prohibit operation of the electrically operated pump for inflation of the inflatable retention member when the present fluid volume is determined to have reached a volume safety threshold. In this second aspect, the pump is a reversible pump, operable also for pumping fluid out from the inflatable retention member for deflation. The reversible pump may be used instead of the electrically operable valve, or in addition to such a valve.

By "continuously determining" is meant a determination which is continuous over a certain period of time, such as during the entire inflation step, during the entire time the pump is active, during the entire irrigation process, at all times, or the like.

Catheters with inflatable retention members, such as an inflatable balloon, are used in many medical procedures where there is a need to retain the catheter in an inserted position during use. For example, such catheters are commonly used when the catheter is intended for insertion into the rectum, so-called rectal catheters, for example for use in anal irrigation systems, and for indwelling urinary catheters, for draining of urine from the bladder.

An appropriate inflation level should be such that the catheter remains in place during use, but should also be such that it does not cause unnecessary discomfort and harm to the user, and in particular the inflatable retention member should not be overinflated to such an extent that there is a risk of burst or explosion of the inflatable retention member. At the appropriate inflation level, the inflatable retention member may also function to prevent the irrigation liquid from leaking out during transanal irrigation.

The appropriate inflation level of the inflatable retention member is difficult to determine beforehand, since it depends on the type of use and the size and type of catheter. It is also dependent on the preferences of the user; some users want the inflatable retention member to be quite large when inflated, whereas other users are more sensitive, and feel discomfort already at relatively small size. Further, this may also vary over time for one and the same user, depending e.g. on the condition of the colon, the general condition of the user, etc.

The present invention allows the user or operator to control the inflation of the inflatable retention member precisely and safely. Since the present fluid volume is determined based on both an estimated inflation fluid volume and an estimated deflation fluid volume, the control is based on a current fluid volume, regardless of whether periods of deflation has occurred during inflation or not. Thus, the volume safety threshold is used in relation to the present fluid volume, which is continuously estimated upwards and downwards, depending on whether the inflatable retention member is inflated or deflated, respectively. This allows for the user to repeatedly inflate and deflate the inflatable retention member, in order to find a comfortable and adequate inflation level.

It has been found that control of the inflatable retention element based on an estimated present fluid volume is much more reliable and accurate than e.g. conventional control, based on pressure measurements. For example, muscle contraction in the rectum may result in high pressure in the retention member even at fairly small volumes. Similarly, in the trachea there are periods of high pressure during the respiratory cycle. Also, at small inflated sizes, the pressure variation is very low, making it very difficult to adequately control the inflation size solely based on the pressure level. There may also be differences in the inflatable retention members, such as differences in material, differences in how the catheter has been previously used, etc. However, by controlling the inflation and deflation based on an estimate of the present fluid volume a very efficient and predictable size can be obtained at various inflation levels, and at the same time it can be ensured that there is no overinflation, ensuring that there is no risk of burst or explosion of the inflatable retention member.

The control may also be realized in a very cost-effective manner, and without the need for costly and complicated sensors and the like.

The present invention is based on the understanding that the volume of an inflatable retention member can be determined with great accuracy based on knowledge of the pressure in the inflatable retention elements, and the fluid flow into and out from the inflatable retention member, which may be estimated based on how and how long the electrically operated pump and the electrically operated valve are activated. Based on this, the controller can determine an inflation flow rate into the inflatable retention member from the electrically operated pump, and a deflation flow rate out from the inflatable retention member through the electrically operated valve. Aggregated and integrated over time, the inflation flow rate can be used to continuously determine an inflation fluid volume, and in the same way the deflation flow rate can be used to continuously determine a deflation fluid volume. A present fluid volume in the inflatable retention member could then continuously be determined as the inflation fluid volume reduced by the deflation fluid volume.

It has been found by the inventors that generally, based on the ideal gas law, this may be expressed as:

$$V = \frac{TR}{P}\left[\sum_{k=0}^{\infty}\int_{t_{k\_inf\_on}}^{t_{k\_inf\_off}}\dot{n}_{inf}(t, \Delta T_t, \Delta P_t)dt - \sum_{m=0}^{\infty}\int_{t_{m\_def\_on}}^{t_{m\_def\_off}}\dot{n}_{def}(t, \Delta T_t, \Delta P_t)dt\right]$$

where:

| Term | Unit | Description |
|---|---|---|
| V | ml | Volume in the inflatable retention member. |
| T | K | Temperature in the retention member. |
| t | s | Time |
| $\Delta T_t$ | K | Temperature difference between inside the retention member and outside the system in the instant t. |
| R | constant | Ideal, or universal, gas constant. |
| P | mbar | Pressure inside the retention member. |
| k | - | Count of the number of inflation periods. |
| $t_{k\_inf\_on}$ | s | Time when an inflation period started. |
| $t_{k\_inf\_off}$ | s | Time when an inflation period stopped. |
| $\dot{n}_{inf}$ | moles/s | Mole flow of fluid moles entering the retention member. |
| m | - | Count of the number of deflation periods. |
| $t_{m\_der\_on}$ | s | Time when a deflation period started. |
| $t_{m\_def\_off}$ | s | Time when a deflation period stopped. |
| $\dot{n}_{def}$ | moles/s | Mole flow of fluid moles leaving the retention member. It's represented by a function of time, difference in temperature and difference in pressure. |
| $\Delta P_t$ | mbar | Pressure difference between inside the retention member and outside the system in the instant t. |

The electrically operated pump may, whenever activated, always be operated at a known speed, such as at a constant speed or a speed determined by a sensor or the like. This makes it possible to determine, e.g. by simple experimental measurements, the air flow provided by the electrically operated pump at different balloon pressure levels.

It has been found that when the pump is operated at a steady state, the air flow (L/min) decreases as the counter pressure (mbar), i.e. the pressure in the inflatable retention member, increases. Differently put, the air flow is initially, when the counterpressure is very low, at its highest, and then decreases rapidly as the counter pressure increases, and then less and less rapidly as the counter pressure continues to increase.

In an embodiment, the electrically operated pump is arranged to operate at a steady state whenever it is operated, i.e. by being supplied by a constant power. Hereby, the pump is generally operated intermittently at binary states, i.e. a steady state "on" state and an "off" state. The binary states may be provided essentially instantaneously, but may also include ramping up and/or down.

However, the pump need not be operated at a constant speed or with a steady state power. The voltage and the current may also be varied during operation of the pump. In such embodiments, the system may further comprise a power sensor for determining at least one of a current and a voltage supplied to the electrically operated pump. The controller may then be arranged to determine the inflation flow rate based on the current and/or voltage supplied to the electrically operated pump, based on input from the power sensor. The relationship between the airflow and the power, at various counter pressure levels, can again easily be determined experimentally. Additionally, or alternatively, the system may comprise a speed sensor, such as a tachometer or encoder, to determine the rotation speed of the pump.

The electrically operated valve may, whenever activated, and possibly after a short ramping up due to inertia, always be fully opened. Thus, the electrically operated valve may be operated intermittently between a fully closed state and a fully opened state. This makes it possible to determine, e.g. by simple experimental measurements, the air flow provided through the electrically operated valve when in an open state at different balloon pressure levels.

It has been found that when the valve is operated to be fully open, the air flow (L/min) increases as the counter pressure (mbar), i.e. the pressure in the inflatable retention member, increases. This relationship has been found to generally follow an exponential growth graph form. Differently put, when the counterpressure is very low, the air flow through the valve would be low, whereas the air flow would increase exponentially as the counter pressure increases. Any ramping up or down of the valve, due to inertia, would be fairly constant, independent of the counterpressure.

In an embodiment, the electrically operated valve is arranged to always be in one of two states, viz. a fully opened state and a fully closed state. Hereby, the valve could be operated intermittently, to switch between the binary states.

However, the valve need not be operated only at two states, and one or more intermediate states, having varying degrees of openness, could also be contemplated. In one embodiment, the degree of openness of the valve may be continuously changed between a fully opened and a fully closed state. In such embodiments, the controller may be capable of determining the degree of openness of the valve, based e.g. on the control signals sent from the controller to the valve. Alternatively, the system may comprise a sensor for determining the degree of openness of the valve. The controller may then be arranged to determine the deflation flow rate based on the degree of openness of the valve. The relationship between the airflow and the degree of openness, at various counter pressure levels, can again easily be determined experimentally.

The pressure in the inflatable retention member can be directly determined by measuring by a pressure sensor. However, the pressure in the inflatable retention member may also be indirectly estimated without any specific pressure sensor. For example, it is possible to estimate the pressure of the inflatable retention member based on the load on the pump.

However, in an embodiment the irrigation system may further comprise a pressure sensor arranged to detect the pressure in the inflatable retention member. Hereby, the accuracy in the determination of the inflation fluid flow and the deflation fluid flow, and thus the inflation fluid volume and the deflation fluid volume, will become even more accurate and reliable.

In an embodiment, the controller is arranged to continuously determine the inflation fluid volume based on the inflation flow rate in combination with the pressure in the inflatable retention member.

Additionally, or alternatively, the controller may be arranged to determine the deflation fluid volume based on the deflation flow rate in combination with the pressure in the inflatable retention member.

The pressure in the inflatable retention member will, during operation, preferably be within the range 1-500 mbar, and preferably within the range 2-300 mbar. As used herein, mbar represents the gauge pressure, or so-called bar(g) or simply barg, i.e. the pressure in bars above ambient or atmospheric pressure. For improved accuracy, the system may further comprise a pressure sensor to determine the ambient pressure.

The pressure sensor may be arranged directly in the inflatable retention member, or in conduit being in direct communication with the inflatable retention member. Thus, in one embodiment, the pressure sensor may be arranged inside the inflatable retention member, and e.g. be connected to the control unit via an electric wire or the like. In another embodiment, the pressure sensor is arranged at a distance from the inflatable retention member, but in the lumen leading to the inflatable retention member, or in a conduit connected to this lumen, but ahead of any obstacles that would affect the pressure, such as the electrically operable valve. It is also possible to use more than one pressure sensor, and to arrange the pressure sensors at different locations.

As discussed above, the volume will also vary in dependence of the temperature. However, since the irrigation system will normally be used at room temperature, and since the temperature inside a human body is essentially constant and equal for all persons, the temperature dependent variation will for all practical use situations be neglectable. However, for rare situations where the temperature has a significant variation, or if there is a need to improve accuracy even further, the irrigation system may also comprise a temperature sensor. In this case, the temperature sensor is preferably arranged on, in or in the vicinity of the inflatable retention member, thereby to sense and determine the temperature on, or in, the inflatable retention member. To further increase accuracy, a temperature sensor may also be arranged to determine the ambient temperature outside the inflatable retention member.

The diameter of the inflatable retention member corresponds to the fluid volume contained in the inflatable retention member. For example, the correlation between the volume (ml) and diameter (mm) of the inflatable retention member has been found to generally follow a log-normal distribution function, where the diameter increases quite rapidly as the volume increases from zero and upwards, but where the increase rate decreases as the volume increases.

The present fluid volume, as determined in the way discussed in the foregoing, is useable for various types of control of the irrigation system.

In an embodiment, the present fluid volume may be used for a safety control of the irrigation system. In particular, the controller may be arranged to prohibit operation of the electrically operated pump when the present fluid volume is determined to have reached a volume safety threshold. Such a volume safety threshold may be determined by simple experimental testing. An inflatable retention member of a catheter may simply be inflated until it bursts, and the volume safety threshold may be determined to be a volume below this bursting volume. However, other ways of determining an adequate volume safety threshold are also feasible.

One and the same volume safety threshold may be used for all catheters useable with the irrigation system. However, in case various catheter types are used together with the irrigation system, different volume safety threshold may be determined for each of the catheter types. The catheter types may e.g. differ in materials used in the catheter, sizes of the catheters and/or the inflatable retention members, etc.

In case different volume safety thresholds are used for different catheter types, the controller of the irrigation system may be arranged to determine the catheter type automatically, and thereby automatically determining or choosing an appropriate volume safety threshold for this catheter type. To this end, the irrigation system may further comprise a property detection sensor arranged to detect a property of the catheter, and wherein the controller is further arranged to set the volume safety threshold based on said property of the catheter. The catheter property may e.g. be catheter type and/or size.

In an embodiment, the property detection sensor comprises a Hall sensor, arranged to determine the presence and/or configuration of magnets in the catheter or tubing connected to the catheter, the magnets being indicative of a size and or type of the catheter. The Hall sensors may e.g. be arranged at, or in the vicinity, of where a catheter or a tube connected to a catheter, is connected to another part of the system, such as a base unit. The Hall sensor may be used to simply determine whether magnets are present or not. However, the Hall sensors may also be used to determine specific patterns or combinations of magnets, specific number of magnets, specific field strengths of the magnets, etc. Hereby, it is possible to distinguish between two, three, four, or even more, types or properties of catheters.

Such determination of a catheter property based on Hall sensor detection is per se known from WO 2020/142185, said document hereby being incorporated in its entirety by reference.

Alternatively, detection of catheter property may occur in other ways, such as by provision of an RFID tag with information on the catheter or tubing connected to the catheter, and an RFID reader connected to the controller. Such a solution is per se known from US 9 138 516, said document hereby being incorporated in its entirety by reference.

In an embodiment the controller may further be arranged to prohibit operation of the electrically operated pump when a pressure measured by the pressure sensor is above a pressure threshold. Hereby, by such an additional safety measure, the safety and reliability of the irrigation system may be improved even further.

The irrigation system may further comprise a memory, storing a set of inflation levels, each level being correlated to a volume level, and a user interface arranged to receive input of a selected inflation level selected form said set of inflation levels, wherein the controller is arranged to operate the electrically operated pump until one of the selected inflation level and the volume safety threshold, has been reached. The controller is hereby arranged to compare the present fluid volume to both the volume safety threshold level and the selected inflation level, and abort pumping as soon as any one of these levels has been reached. I

Operation of the pump by the controller may be such that the controller automatically continues to operate the pump until one of the levels has been reached after input from an interface control, such as depression of a start button. However, the controller may alternatively be arranged to operate the pump as long as the interface control is activated, such as during depression of a pumping button. In this case, the pumping will be aborted either when the pump button is released, or when one of the levels has been reached, regardless of whether the pump button is then still activated and depressed or not.

Due to the above-discussed determination of the present fluid volume based on an inflation fluid volume and a deflation fluid volume, the present fluid volume will always correspond to the actual fluid volume in the inflatable retention element, regardless of whether inflation occurs in a single inflation action, in a plurality of inflation actions, or in a plurality of inflation actions with deflation actions intervened therebetween.

Various number of predetermined selectable inflation levels may be provided, such as 3 or more, 5 or more, etc. In an embodiment, the number of selectable inflation levels is 5-10. Hereby, different inflation levels may be selected in dependence on the particular user, the type and size of catheter being used, the present condition of the user, etc.

Preferably, the plurality of predetermined inflation levels comprises at least 3 different inflation levels, and preferably at least 5 different inflation levels. The inflation levels can, in the case of three levels, e.g. be denominated 1, 2 and 3; A, B and C; S, M and L; or the like, or be illustrated by schematic representations of balloons in three different sizes. The user may select a desired inflation level in various ways, such as by using a button or key dedicated for the specific inflation level, by selecting a level in a menu or the like, by using "+" and "-" buttons to increase and decrease the present level, etc.

The inflation/deflation control will ensure proper inflation even during contextual variations, such as variations in the counter pressure of the colon.

The control of the present invention is also relatively simple to realize, install and operate, thereby making the resulting method/system cost-efficient and user friendly.

An electrically operated pump is of great advantage, in particular in irrigation systems, since it can be operated very easily, which is particularly advantageous for users with reduced dexterity. If the user lacks strength in their hands it may be easier for him/her to operate an electrically operated pump rather than squeezing e.g. a foil-pump. The electrically operated pump can also easily be adjusted and customized for different types of use, for different types of users, etc.

In an embodiment the electrically operated pump is a diaphragm pump. It has been found that diaphragm pumps are cost-efficient and reliable, and also relatively simple to analyze to determine a correlation between the supplied power and the resulting output air flow.

The irrigation system as discussed above and, in the following, may be used for rectal and/or stomal irrigation.

The system of the present invention is particularly useful for use in rectal or anal irrigation system, as well as in stomal irrigation. However, the method/system of the present invention is also useable in other catheter systems having inflatable retention members, such as in urinary catheter systems, in endotracheal intubation systems etc.

In rectal or anal irrigation, an irrigation liquid is introduced into the rectum and lower intestine of a patient in order to induce bowel movement. The need for such a procedure typically arises in patients suffering from certain physical ailments in which voluntary bowel control is impaired or when the bowel needs to be cleaned before e.g. a coloscopy or a surgical operation. To this end, irrigation systems may be used e.g. by people suffering from spinal cord injuries, spina bifida or multiple sclerosis. For such users, irrigation may improve quality of life by preventing constipation, reducing time spent for bowel emptying procedures, reducing fecal incontinence, and by increasing independency and quality of life in general.

The irrigation system of the present invention is preferably portable, of limited size and relatively simple to use and control, also for user's having reduced dexterity. This makes it very well suited for self-administration, i.e. when irrigation is performed outside medical attendance premises, such as in the patient's home, and is performed by the patient himself. Further, portability of the irrigation system is of advantage for disabled persons who are not hospitalized or bed-ridden if they are to live as normal a life as possible. This is particularly important if they travel away from their home, for instance, to someone else's home, or if they stay in a hotel. In this situation, they need to be able to deal with their bowel function easily.

Thus, the new irrigation system facilitates operation, in particular for users having reduced dexterity. The whole irrigation procedure hereby becomes easier, faster and easier to control, and at the same time the overall safety is increased, and in particular the risk of explosion or bursting of the inflatable retention member is prevented.

In an embodiment, the catheter is a rectal catheter, and wherein the catheter system is a rectal, anal or transanal irrigation system, said system further comprising:

    a reservoir for an irrigation liquid;
    tubing providing fluid communication between said reservoir and said catheter; and
    an electrically operated pump for directly or indirectly pumping irrigation liquid from the reservoir to the probe through said tubing, wherein said electrically operated pump is either the same as the electrically operated pump for pumping a fluid for inflation of the inflatable retention member, or a second pump.

In a preferred embodiment, the electrically operated pump is arranged for indirect pumping of irrigation liquid from the reservoir to the probe. Hereby, the pump pumps a gas, such as ambient air, into the reservoir, whereby the increase in pressure in the reservoir forces an irrigation liquid contained therein to be pumped out.

In an embodiment, the irrigation system further comprises a control unit with a housing, said housing enclosing said controller, said electrically operated pump, and said electrically operated valve. In an embodiment, the control unit may further be integrated with the reservoir. For example, the control unit housing may form a base or a platform on which the reservoir is arranged. The reservoir may be fixedly or releasably connected to the control unit housing.

The control unit further preferably comprises control elements for operation of the irrigation system. Additionally, or alternatively, such control elements may be provided on a remote control, wirelessly connected to the control unit.

Preferably, the control unit and/or remote control comprises control elements for pumping of irrigation liquid, and for inflation and deflation of the inflatable retention member. The control unit and/or remote control may also comprise control elements operable to set the desired inflation level.

The control unit and/or remote control may further comprise a display. The display may be used to assist in and confirm the selection of the desired inflation level. Thus, the display may be used to show different available inflation levels for the selection, and may also be used to show the selected inflation level, once the selection has been made.

Further, the display may be used to display information to the user about the progress of the irrigation procedure, such as volume that has been pumped, present flow rate, time elapsed from the start of the procedure, or estimated time left, etc. Further, the display may be used to guide the user about what choices in terms of settings and the like that are needed, the present function of the control elements, etc.

Still further, the display may be a touch screen, useable also for inputting data into the system. For example, the control elements may be realized as areas on the touch screen. If the control unit is connected wirelessly to a remote control or other remote unit, the display on this device may be used to display information. Thus a display on a remote control or other remote unit may be used to replace the display on the control unit, or to complement a display on the control unit.

By the use of a remote control, the control unit may e.g. be placed on the floor, or in any other resting position, and instead be operated through the remote control during irrigation. This facilitates handling of the irrigation system and affords the user an increased freedom in terms of how to use the system. The remote control may be a dedicated remote control, specifically arranged to control the irrigation system. However, the remote control may also be a common wireless device, capable of transmitting wireless control signals to a receiver in the control unit. In one preferred embodiment, the remote control is a mobile telephone, and preferably a smart phone. Additionally or alternatively, the remote control may be a laptop computer or a tablet computer. Hereby, a special application may be downloaded to the smart phone/laptop/tablet computer, providing a suitable interface for the device, and enabling it to send appropriate control signals to the control unit.

Inflation of the inflatable retention member may be performed in various ways. In one embodiment, inflation is started by the user, e.g. by pushing a control element, such as a button or key, whereupon the inflation automatically proceeds until the selected inflation level has been reached, or alternatively when the volume safety threshold has been reached. Alternatively, inflation only occurs when a dedicated control element such as a button or key is depressed, thereby functioning as a dead man's handle, immediately returning to a deactivated state in which the electrically operated pump is controlled not to pump, when manual operation of the control element is aborted. Thus, the inflation pumping can at any time be aborted by the user or operator. However, when, at any time, pumping is resumed, the automated pumping continues from the state where it was aborted. Thus, the controller continues monitoring the present fluid volume. Thus, the controller assures that pumping will be stopped as soon as the selected inflation level or the volume safety threshold level has been reached, or the control element for pumping is released. If the pumping has been aborted due to release of the control element, the pumping will be resumed, and the process be continued as before, when the control element is once again activated.

Similarly, the control element(s) for pumping the irrigation liquid may be arranged as separate control element(s), and may also be assigned to a dead man's functionality.

By means of this dead man's handle functionality it is ensured that pumping is immediately aborted when the control element is released. This means that the pumping action is stopped immediately when the control element is released, regardless of whether this release is intentional or by accident. For example, the pumping will stop immediately if the control element is accidentally dropped. Further, stopping by releasing is a very intuitive and quick operation method, which is both ergonomically favorable and fast. Thus, at least one, and preferably both of the control element(s), for controlling pumping of the irrigation liquid and for pumping fluid for inflation of the inflatable member, preferably functions as a dead man's handle, thereby immediately returning to a deactivated state in which the electrical pump is controlled not to pump, when manual operation of the control element is aborted.

The control elements are operable by applying a predetermined condition to bring the control element into the activated state, and preferably at least one of depression, twisting, rotating, pulling and pushing. If a control button is used, e.g. realized as an area on a touch screen, the predetermined condition is preferably depression, so that the control button is activated by depressing it, and deactivated by releasing it. However, alternative types of control elements, such as rotatable knobs, switching levers and the like may also be used. An automatic return to the deactivated state when the predetermined condition ceases can e.g. be obtained by a spring, an elastic element or the like, operable to provide a counterforce to the force applied by the manual operation. The control elements, such as control buttons, may be arranged on the surface of the housing. The control elements may e.g. be realized as areas on a touch screen.

The control unit preferably comprises at least two control elements, such as control buttons, and preferably at least three control elements. Two, or preferably three, control elements enable a very easy manipulation of the control unit, and at the same time provides numerous input alternatives. It is further preferred that at least one of the control element(s) is a multi-purpose control element having different functions in different operation states. Hereby, the control elements can e.g. be assigned to control different functions during initiation/set-up and during operative use.

One or several control elements may also be arranged separated from the control unit, and may e.g. be connected with the control unit by means of wire, thereby be physically connected to the electric pump etc. Alternatively, the control elements may be arranged on a remote control, which is wirelessly connected to the rest of the irrigation system. The remote control can e.g. be at least one of: a smart phone, a tablet computer and a laptop computer. It is also possible to combine a control unit with integrated control elements and a remote control, whereby the user may choose whether to use the integrated control unit or the remote control, or both, for controlling the irrigation process.

The wireless communication between the control unit and a remote control or a remote unit may be obtained in many ways, as is per se well known in the art, such as by infrared light (IR), ultrasonic communication, radio frequency (RF) communication such as Bluetooth, etc.

The control unit is further preferably provided with a battery for driving the controller, the electrically operated pump(s) and the electrically operated valve.

Preferably, all components of the irrigation system are individually exchangeable, so that e.g. the probe/catheter can be exchanged frequently, and typically be used only once, whereas other parts of the system, such as the control unit, the electrical system and the irrigation liquid reservoir can be used for months or even years.

The irrigation system of the present invention comprises relatively few and uncomplicated components, which may be reused for a long time, which makes the irrigation system relatively easy and cost-efficient to produce. Further, the irrigation system lends itself well for automated or semi-automated manufacturing.

The irrigation system of the present invention is also highly suitable for self-administration of the irrigation liquid. The control elements on the control unit also make it easy to access the pump(s) with one hand only, and to switch between different pumping modes etc. Typically, with this arrangement, it is e.g. possible to operate the irrigation system with one finger, e.g. the thumb. This provides a very convenient and precise controllability of the irrigation system.

The inflatable retention member is preferably inflated with a gas, and most preferably air. However, the inflatable retention member may also be inflated with a liquid, such as water. Thus, the pump may alternatively be arranged to pump any other such inflation fluids, either directly or indirectly, to the inflatable retention member.

These and other aspects of the invention will be apparent from and elucidated with reference to the embodiments described hereinafter.

BRIEF DESCRIPTION OF THE DRAWINGS

For exemplifying purposes, the invention will be described in closer detail in the following with reference to embodiments thereof illustrated in the attached drawings, wherein:

Fig. 1 illustrates an irrigation system in accordance with an embodiment of the present disclosure;
Fig. 2 schematically illustrates a side view of a container useable in embodiments of the present disclosure;
Fig. 3 schematically illustrates a side view of a container useable in another embodiment of the present disclosure;
Fig. 4 is a schematic block diagram of an irrigation system in accordance with an embodiment of the present disclosure;
Fig. 5 is a schematic block diagram illustrating the parts the irrigation system in Fig. 4 useable for inflation and deflation control in more detail;
Fig. 6. is a flow chart illustrating a method for controlling inflation in accordance with an embodiment of the present disclosure;
Fig. 7 is a diagram illustrating a correlation between diameter and volume for an inflatable retention member;
Fig. 8 is a diagram illustrating a correlation between flow rate and a counterpressure in an inflatable retention member;
Fig. 9 is a diagram illustrating a correlation between an operational current for an electric pump and a flow rate; and
Fig. 10 is a diagram illustrating a correlation between valve flow rate and pressure in the inflatable retention member.

DETAILED DESCRIPTION OF CURRENTLY PREFERRED EMBODIMENTS

The irrigation system of the present disclosure is particularly useful for rectal and transanal irrigation. However, the irrigation system may also be used in other types of medical irrigation procedures. The following discussion is, in particular, concerned with the preferred field of use, rectal and transanal irrigation systems, even though the disclosure is not limited to this particular type of irrigation systems.

It is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length and width of the container, etc.

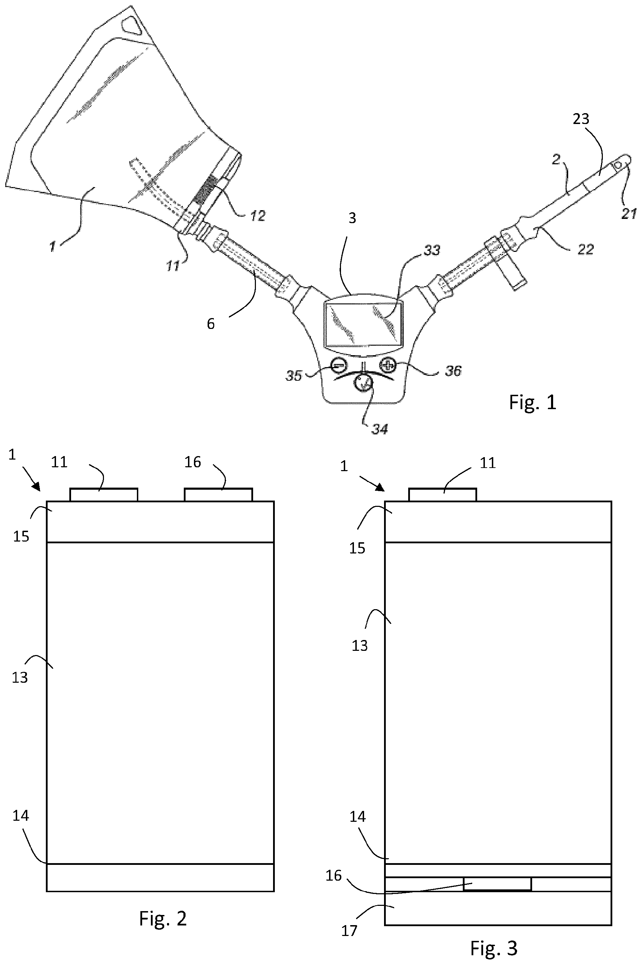

Fig. 1 discloses an irrigation system according to a first exemplary embodiment, comprising a container 1 for an irrigation liquid, a catheter or probe 2 for arrangement in a user, and a control unit 3.

The container may be realized in various ways. For example, the container may be formed by a rigid, semi-rigid or flexible material. In case a semi-rigid or flexible material is used, the container may be collapsible or foldable, to make the irrigation system more compact prior to use. The container is preferably provided with an opening, closed by a closure 11, for filling of the reservoir. Tubing connecting the container to the rest of the irrigation system may be provided through the closure 11, or through other access points on the container.

In order to render the irrigation system as portable as possible, the container preferably has a capacity of less than 5 liters, more preferred less than 3 liters and most preferred less than 2 liters. If, however, the system is to be used for repeated irrigation, a larger capacity container may be necessary.

The container may comprise an overpressure release valve, to release pressure over a predetermined maximum pressure to be allowed. Further, the container may also, optionally, comprise a filter 12, such as a hydrophobic filter, which is impermeable to the irrigation liquid, but which allows air to enter the reservoir but not escape the container. Such a filter ensures that the container maintains its shape when irrigation liquid is being pumped out from the container.

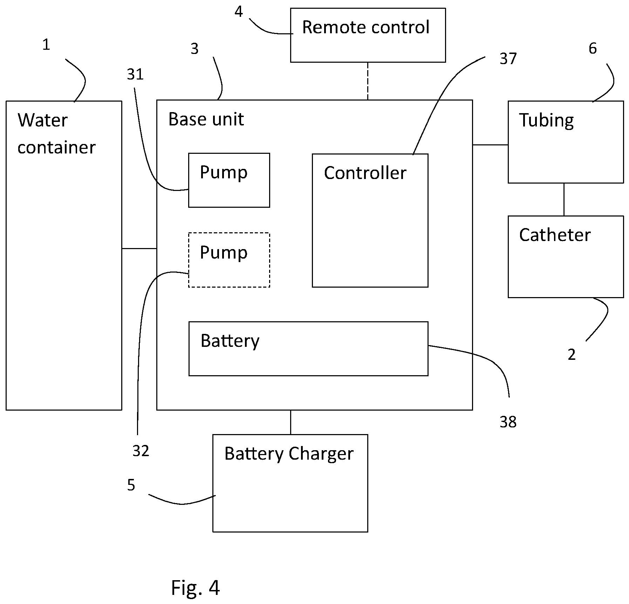

A pump 31, best seen in Fig. 4, may be used to pump air into the container for, indirectly, pumping liquid out of the container. Alternatively, the pump may directly pump liquid out of the container.

The catheter/probe 2 is provided with a retention member, such as an inflatable balloon 23, in the vicinity of an insertable end 21, for fixing the catheter in a body cavity.

The pump 31 used for indirect or direct pumping of irrigation liquid from the water container may also be used for pumping air or other fluids into the inflatable retention element. The pump 31 may e.g. be arranged to pump air, and valves may be used to direct the pumped air into the water container and the inflatable retention member, respectively. However, alternatively, a second pump 32 may be provided to pump air or other fluids into the inflatable retention member.

In a preferred embodiment, the pump 31 is arranged for indirect pumping of the irrigation liquid. Hereby, the pump pumps a fluid, preferably a gas such as air, through a conduit to the reservoir. Thereby, pressure increases in the reservoir to pump irrigation liquid through another conduit to the control unit. This conduit preferably passes through an electrically operable valve and optionally a flow sensor (not shown) and continues to the probe, for dispensing the irrigation liquid to the user. The valve is preferably connected to the controller 37 (see Fig. 4), so that the controller may control the degree of openness of the valve. In case a flow sensor is provided, the input from the flow sensor may be used by the controller to regulate the valve.

The valve may be an on/off valve, arranged only to assume a fully opened or fully closed state. However, the valve may also provide intermediate positions, and may e.g. be gradually controllable between these end states. Such an electrically operable valve can be realized in many ways, as is per se well-known in the art. For example, the electrically operable valve may be a clamp or pinch valve, providing a controllable clamping/pinching action on a tube leading between the electrical pump and the probe. For example, the valve may be of the type disclosed in US 2006/0114148 by the same applicant, said document hereby being incorporated in its entirety by reference.

The one or more pumps are preferably electrically operated pump. The pumps may e.g. be diaphragm pumps, which is generally non-reversible, but other pump types, such as peristaltic pumps, which may be reversible, are also feasible.

The reservoir/container 1 may be realized in various ways. For example, the reservoir may be formed by a rigid, semi-rigid or flexible material. In case a semi-rigid or flexible material is used, the reservoir may be collapsible or foldable, to make the irrigation system more compact prior to use. The reservoir is provided with an opening, closed by a lid 11, for filling the reservoir. Tubing 6, connecting the reservoir to the rest of the irrigation system, may be provided through the lid 11 or through other access points on the reservoir.

The catheter 2 is here embodied as a rectal catheter. The probe is provided with an inflatable retention member 23, such as an inflatable balloon, for fixing the catheter in a body cavity. The inflatable balloon preferably protrudes as a toroidal shape when inflated, and is essentially flush against the wall of the catheter when deflated. The inflatable retention member may also be referred to as a balloon, and is arranged close to the insertable tip 21, but at some distance from the end. Between the tip and the balloon, an opening for dispensing liquid such as irrigation liquid to, or draining liquid from the body, may be provided. The inflatable retention member may be made of any suitable material, such as PVC, latex, TPE or PU. However, other materials providing similar properties can likewise be used.

Further, the probe may be provided with a rearward enlarged part 22, providing an abutment to hinder too deep insertion. The catheter/probe is preferably provided with two lumens - one lumen for transfer of irrigation liquid through the probe, for discharge at the forward end, and one lumen for inflation and deflation of the balloon.

The probe may be of the type disclosed in WO 2014/154635, said document hereby being incorporated in its entirety by reference.

A control unit 3 may be provided. The control unit may be realized as a unitary, handheld unit, as in the illustrative example of Fig. 1. However, the control unit may alternatively be arranged as a base unit, e.g. arranged to be placed on the ground or floor during use.

As one embodiment, the reservoir may be a collapsible reservoir of the type disclosed in US 2015/0335529, said document hereby being incorporated in its entirety by reference.

In order to render the irrigation system as portable as possible, the container preferably has a capacity of less than 5 liters, more preferred less than 3 liters and most preferred less than 2 liters. However, when the system is to be used for repeated irrigation, a larger capacity container may be necessary.

The irrigation liquid can be any liquid which is capable of irrigating the body cavity of interest. In order to stimulate bowel movements, suitable irrigation liquids include water, hypertonic aqueous salt solutions, solutions or suspensions of cathartic agents, such as bisacodyl or phenolphthalein, and mineral oil.

Referring to Figs. 2 and 3, the container 1 may comprise a flexible side wall member 13, a rigid bottom portion 14, a rigid top portion 15, an inlet opening 11, and an outlet opening 16.

In the embodiments illustrated in Figs. 2 and 3, when assembled, the bottom portion 14 is arranged at a first open end of the side wall member 13 such that it seals the first open end. The rigid top portion 15 is arranged at a second open end of the side wall member 13 such that it seals the second open end.

In the embodiment of Fig. 2, both a filling opening 11 and an outlet opening 16 are provided in the rigid top portion. However, alternatively, as illustrated in Fig. 3, the outlet opening may instead be provided in the rigid bottom portion 14, which may e.g. be docked into a base portion 17. In such embodiments, the base portion 17 may comprise the controller 3, then realized as a base unit. The side wall 13 may be made from a flexible sheet material.

The control unit may be arranged connected to the container and the probe through tubing. Alternatively, the control unit may be directly connected to, and possibly even integrated with, the water container.

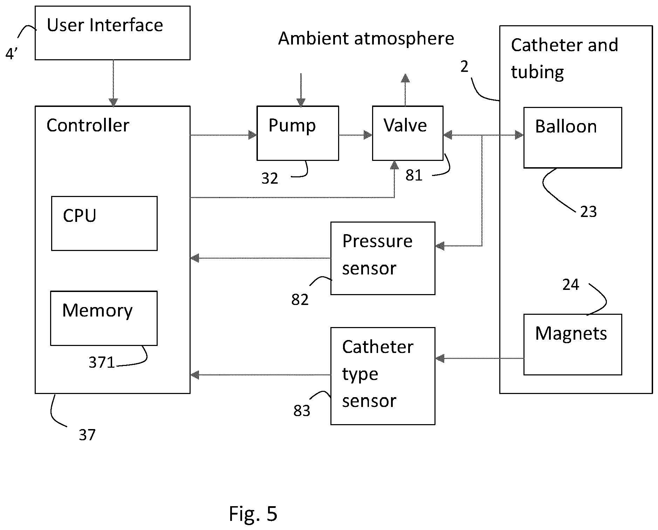

The first electrically operated pump 31, as well as the optional second electrically operated pump 32, for pumping irrigation liquid and inflation fluid may be provided within the control unit 3, as the illustrative example of Figs. 4 and 5, but may also be arranged outside the bounds and housing of the control unit.

The control unit may comprise a display 33, and a user interface for providing input to the system, e.g. including one or several control elements. In the example of fig. 1, three control elements 34, 35 and 36 are provided. The control elements are preferably realized as depressible control buttons. The control unit is preferably waterproof. The control elements may thus be realized with thick pliable plastic or the like, designed to withstand many pushes.

However, alternatively, as in the illustrative example of Fig. 4, the control elements may instead be provided on a remote control 4, and may e.g. be realized as sensor areas on a touch screen.

The control elements 35 and 36 may here be used to activate the pump(s) for inflation/deflation of the inflatable retention member, transfer of irrigation liquid through the probe for irrigation (control element 36), releasing overpressure and/or draining the system of remaining liquid (control element 35). Separate control elements may also be provided for irrigation and inflation, so that inflation and deflation of the retention member may take place independently of the irrigation, and e.g. simultaneously.

The control unit or base unit comprises a controller 37. The control unit or base unit may further comprise one or more of the above-discussed pumps 31, 32 and a battery 38.

The controller 37 may be a microprocessor, MCU, comprising one or several central processing units, CPUs. The controller may also comprise two or more MCUs. However, the controller may also be realized in other ways, as is per se known in the art. Further, the controller 37 is preferably provided with one or several memories, either arranged integrated with the controller, or arranged as a separate component connected to the controller. The memory may be a ROM memory, such as an EPROM or EEPROM, or a RAM memory, such as Flash memory. However, many other types of memories may also be used, as is per se well known in the art.

Further, the controller is optionally connected to a wireless transceiver, which is adapted to transmit and receive data from the remote control 4, and/or other remote units. Hereby, the remote control may provide control data to the controller 37, for remote control of the control unit. Additionally or alternatively, the controller may transmit data about the irrigation procedure to the remote control or any other remote unit. The remote control 4 may e.g. be a smart phone or the like.

The battery 38 may be a rechargeable battery, chargeable through an external battery charger 5. Charging may e.g. occur through inductive charging from the charging station 5, or as direct charging from a connected electric conductor.

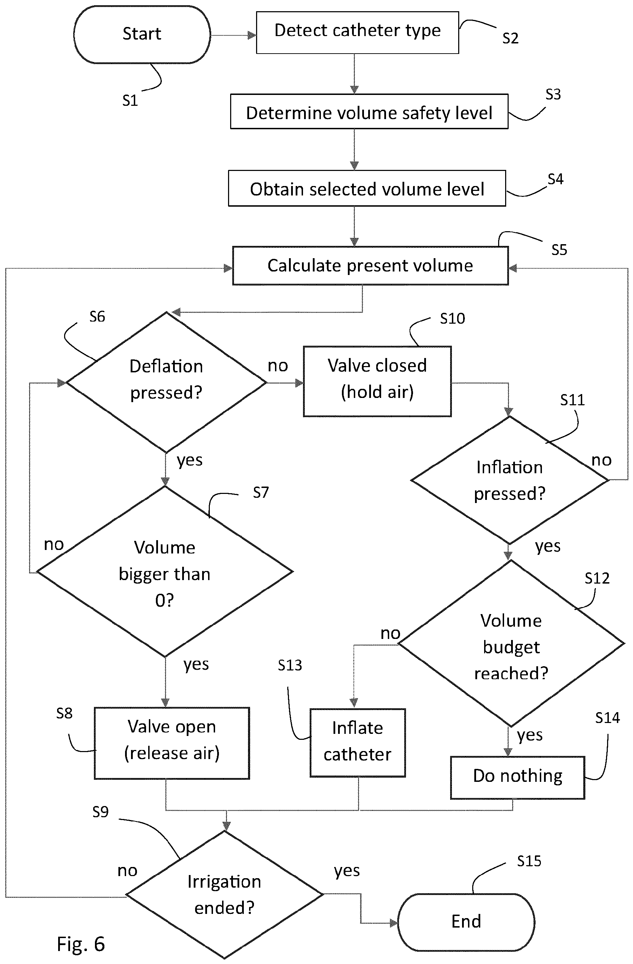

The part of the system handling the inflation and deflation of the inflatable retention member, and the operational procedure for handling inflation and deflation, will now be discussed in more detail with reference to Figs. 5 and 6.

A pump 32 is arranged to pump air through a conduit to the probe for inflation of the inflatable retention member 23. In the illustrated embodiment, different pumps are used for irrigation and inflation. However, as already discussed, a single pump may be used for both purposes.

The pump 32 is controlled by the controller 37. Hereby, the controller may start and stop the pump, thereby starting and stopping the inflation, and may also possibly control the operating speed of the pump.

The pump 32 pumps air (or other inflation fluids) through an electrically controllable valve 81 and into the inflatable retention member 23. The valve 81 can be controlled by the controller 37 to assume a fully opened state and a fully closed state, and preferably also intermediate states. In an embodiment, the valve is continuously controllable between any state between the fully closed and opened states. When opened, the valve releases air from the inflatable retention member 23 into the ambient atmosphere, for deflation of the inflatable retention member.

A pressure sensor 82 is further arranged to measure the pressure in the balloon 23. The pressure sensor may be arranged inside the balloon, or in a conduit being in direct communication with the inflatable retention member. In the illustrative example, the pressure sensor is arranged in a conduit leading from the valve 81 to the balloon 23.

The pressure sensor may be any type of per se known pressure sensors. Preferably, the pressure sensor is a gauge pressure sensor, measuring the pressure relative to atmospheric pressure, and may e.g. be a piezoresistive sensor, such as a piezoresistive strain gauge, a capacitive sensor, an electromagnetic sensor, an optical sensor or the like. However, other types of pressure sensors may also be used.

The measurement output from the sensor 82 is forwarded to the controller 37, which may then control the operation of the pump 32 in accordance with this, as will be discussed in more detail in the following.

The controller 37 is arranged to determine an inflation flow rate into the inflatable retention member from the electrically operated pump 32, and a deflation flow rate out from the inflatable retention member through the electrically operated valve 81, to continuously determine an inflation fluid volume based on the inflation flow rate, a deflation fluid volume based on the deflation flow rate, and to continuously determine a present fluid volume in the inflatable retention member as the inflation fluid volume reduced by the deflation fluid volume.

The controller 37 may further control the electrically operated pump 32 and/or the electrically operated valve 81 based on the determined present fluid volume. For example, the controller 37 may be arranged to prohibit operation of the electrically operated pump when the present fluid volume is determined to have reached a volume safety threshold.

One and the same volume safety threshold may be used for all catheters useable with the irrigation system. However, in case various catheter types are used together with the irrigation system, different volume safety threshold may be determined for each of the catheter types. The catheter types may e.g. differ in material used or size of the catheter and/or the inflatable retention member, etc.

In case different volume safety thresholds are used for different catheter types, the controller of the irrigation system may be arranged to determine the catheter type automatically, and thereby automatically determining or choosing an appropriate volume safety threshold for this catheter type. To this end, the irrigation system may further comprise a property detection sensor 83, arranged to detect a property of the catheter, and wherein the controller is further arranged to set the volume safety threshold based on said property of the catheter. The catheter property may e.g. be catheter type and/or size.

In an embodiment, the property detection sensor 83 comprises a Hall sensor, arranged to determine the presence and/or configuration of magnets 24 in the catheter or in the tubing 6 connected to the catheter, the magnets being indicative of a size and or type of catheter. The Hall sensors may e.g. be arranged at, or in the vicinity, of where a catheter, or a tube connected to a catheter, is connected to another part of the system such as a base unit. The Hall sensor may be used simply to determine whether magnets are present or not. However, the Hall sensors may also be used to determine specific patterns or combinations of magnets, specific number of magnets, specific field strengths of the magnets, etc. Hereby, it is possible to distinguish between two, three, four, or even more, types or properties of catheters. Alternatively, detection of catheter property may occur in other ways, such as by provision of an RFID tag with information on the catheter or on the tubing connected to the catheter, and an RFID reader connected to the controller.

In an embodiment the controller may further be arranged to prohibit operation of the electrically operated pump when a pressure measured by the pressure sensor 82 is above a pressure threshold.

The irrigation system may further comprise a memory 371, e.g. integrated in the controller 37, storing a set of inflation levels, each level being correlated to a volume, and a user interface 4' arranged to receive input of a selected inflation level selected form said set of inflation levels. The controller 37 may then be arranged to operate the electrically operated pump until one of the selected inflation levels or the volume safety threshold has been reached. To this end, the controller 37 is hereby arranged to compare the present fluid volume to both the volume safety threshold level and the selected inflation level, and abort pumping as soon as any one of these levels has been reached. Preferably, the plurality of predetermined inflation levels comprises at least 3 different inflation levels, and preferably at least 5 different inflation levels.

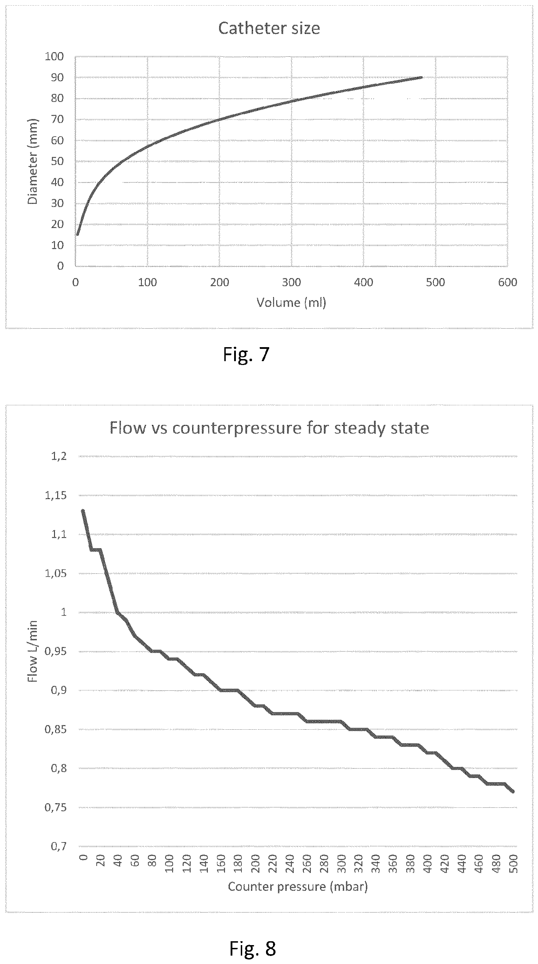

The size of the inflatable retention member generally corresponds to the fluid volume contained in the inflatable retention member. Such a correlation for an exemplary catheter is illustrated in Fig. 7. In this example, the correlation between the volume (ml) and diameter (mm) of the inflatable retention member was found to generally follow a log-normal distribution function, where the diameter increases quite rapidly as the volume increases from zero and upwards, but where the increase rate decreases as the volume increases.

The electrically operated pump may, whenever activated, always be operated at a constant speed, and e.g. supplied by a constant voltage and current. This makes it possible to determine, e.g. by simple experimental measurements, the air flow provided by the electrically operated pump at different balloon pressure levels.

It has been found that when the pump is operated at a steady state, the air flow (L/min) decreases as the counter pressure (mbar), i.e. the pressure in the inflatable retention member, increases. Such a relationship for an exemplary catheter is illustrated in Fig. 8. The relationship generally follows an exponential graph with a decreasing form. Differently put, the air flow is initially, when the counterpressure is very low, at its highest, and then decreases rapidly as the counter pressure increases, and then less and less rapidly as the counter pressure continues to increase.

In an embodiment, the electrically operated pump is arranged to operate at a steady state whenever it is operated, i.e. by being supplied by a constant power. Hereby, the pump is generally operated intermittently at binary states, i.e. a steady state "on" state and an "off" state.

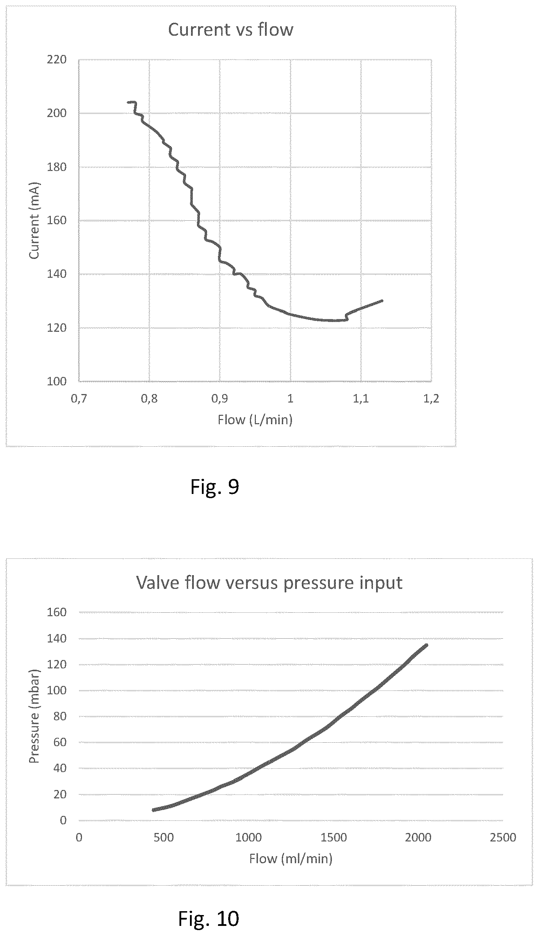

However, the pump need not be operated at a constant speed or with a steady state power. The voltage and the current may also be varied during operation of the pump. In such embodiments, the system may further comprise a power sensor for determining at least one of a current and a voltage supplied to the electrically operated pump. In Fig. 9 a relationship between current (mA) and Flow (L/min) for an exemplary pump is illustrated. The controller may then be arranged to determine the inflation flow rate based on the current and/or voltage supplied to the electrically operated pump, based on input from the power sensor. The relationship between the airflow and the power, at various counter pressure levels, can again easily be determined experimentally.

The electrically operated valve may, whenever activated, always be fully opened. Thus, the electrically operated valve may be operated intermittently between a fully closed state and a fully opened state. This makes it possible to determine, e.g. by simple experimental measurements, the air flow provided through the electrically operated valve when in an open state at different balloon pressure levels.

It has been found that when the valve is operated to be fully open, the air flow (L/min) increases as the counter pressure (mbar), i.e. the pressure in the inflatable retention member, increases. Such a relationship for an exemplary catheter is illustrated in Fig. 10. This relationship has been found to generally follow an exponential growth graph form. Differently put, when the counterpressure is very low, the air flow through the valve would be low, whereas the air flow would increase exponentially as the counter pressure increases.

In an embodiment, the electrically operated valve is arranged to always be in one of two states, viz. a fully opened state and a fully closed state. Hereby, the valve could be operated intermittently, to switch between the binary states.

However, the valve need not be operated only at two states, and one or more intermediate states, having varying degrees of openness, could also be contemplated. In one embodiment, the degree of openness of the valve may be continuously changed between a fully opened and a fully closed state. In such embodiments, the controller may be capable of determining the degree of openness of the valve, based e.g. on the control signals sent from the controller to the valve. Alternatively, the system may comprise a sensor for determining the degree of openness of the valve. The controller may then be arranged to determine the deflation flow rate based on the degree of openness of the valve. The relationship between the airflow and the degree of openness, at various counter pressure levels, can again easily be determined experimentally.

Inflation of the inflatable retention member may be performed in various ways. In one embodiment, as illustrated in Fig. 6, inflation is started at S1, e.g. by the user pushing a control element, such as a button or key.

In a subsequent step S2, the catheter type may be determined. This can, as discussed in the foregoing, e.g. be made by use of a catheter type sensor. However, in other embodiments this step may also be omitted.

Thereafter, in step S3, a volume safety level is determined. In some embodiments, there may be only one volume safety level, which is used for all catheters. This is particularly of relevance if the irrigation system only uses one type of catheter. In such embodiments, the only available volume safety level is determined to be used. However, if more than one type of catheter is available, such as catheters of different sizes, different volume safety levels may be used and assigned to different catheters. Determination of which volume safety level may then e.g. be made in dependence of the detected catheter type in step S2.

In step S4 a selected volume level may then be obtained, e.g. through input from a user interface.

All of the steps S2-S4 are optional, and one or more of these steps may be omitted.

In step S5, a present fluid volume is calculated, based on the pressure, the operation time of the electrically operated pump and valve, etc, as discussed in the foregoing.

It is then determined in step S6 whether a control element for deflation has been activated.

If activation of deflation control has been detected, it is determined in step S7 whether the present fluid volume is greater than zero, and if so, the valve is opened in step S8 for release of air from the inflatable retention element.

Alternatively, if it is determined that there is no activation of a deflation control, the valve is closed or maintained closed, step S10, and it is then determined whether a control element for inflation has been activated, step S11.

If activation of an inflation control has been detected, it is determined in step S12 whether the present fluid volume is greater than a volume budget, step S12. The volume budget is here considered to be the smallest of the determined volume safety level and the selected volume level. Alternatively, separate comparison steps for comparison with both the volume safety level and the selected volume level independently could also be made. In case the volume budget has not been reached, the catheter retention member is inflated, step S13, whereas if it is determined that the volume budget has been reached, the electrically operated pump is kept idle, step S14.

If it is determined in step S11 that there is no activation of any inflation control element, the process may return to step S5.

After steps S8, S13 and S14, respectively, the process may continue to step S9, where it is determined whether the irrigation procedure has been ended. If this is the case, the process ends, step S15, but otherwise, the process may return to step S5.

Hereby, the process may be repeated and iterated continuously until the irrigation procedure has been ended.

Notably, the sequence of the steps discussed in the foregoing may be different than the one discussed in relation to the exemplary embodiment of Fig. 6. For example, step S11 may be performed prior to step S6, step S12 may be performed earlier in the process, such as immediately following step S5, etc.

The person skilled in the art realizes that the present invention by no means is limited to the preferred embodiments described above. On the contrary, many modifications and variations are possible within the scope of the appended claims. For example, the inflation flow rate and deflation flow rate could be determined as estimated in various ways. The irrigation system could also be used for other purposes than rectal or transanal irrigation, such as other forms of irrigation, or for other medical procedures. Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

**Claims**

1. An irrigation system for medical use comprising:

   a catheter having an inflatable retention member;
   an electrically operated pump for pumping a fluid into the catheter for inflation of the inflatable retention member;
   an electrically operated valve for deflation of the inflatable retention member;
   and
   a controller for controlling the electrically operated pump and the electrically operated valve, the controller arranged to determine an inflation flow rate into the inflatable retention member from the electrically operated pump, and a deflation flow rate out from the inflatable retention member through the electrically operated valve, to continuously determine an inflation fluid volume based on the inflation flow rate, and a deflation fluid volume based on the deflation flow rate, to continuously determine a present fluid volume in the inflatable retention member as the inflation fluid volume reduced by the deflation fluid volume, and to prohibit operation of the electrically operated pump when the present fluid volume is determined to have reached a volume safety threshold.

2. An irrigation system for medical use comprising:

   a catheter having an inflatable retention member;
   an electrically operated reversible pump for pumping a fluid into the catheter for inflation of the inflatable retention member and out from the catheter for deflation of the inflatable retention member; and
   a controller for controlling the electrically operated pump, the controller arranged to determine an inflation flow rate into the inflatable retention member from the electrically operated pump, and a deflation flow rate out from the inflatable retention member through the electrically operated pump, to continuously determine an inflation fluid volume based on the inflation flow rate, and a deflation fluid volume based on the deflation flow rate, to continuously determine a present fluid volume in the inflatable retention member as the inflation fluid volume reduced by the deflation fluid volume, and to prohibit operation of the electrically operated pump for inflation of the inflatable retention member when the present fluid volume is determined to have reached a volume safety threshold.

3. The irrigation system of claim 1 or 2, further comprising a property detection sensor arranged to detect a property of the catheter, and wherein the controller is further arranged to set the volume safety threshold based on said property of the catheter.

4. The irrigation system of claim 3, wherein the property detection sensor comprises a Hall sensor, arranged to determine the presence and/or configuration of magnets in the catheter or tubing connected to the catheter, the magnets being indicative of a size and/or type of the catheter.

5. The irrigation system of any one of the preceding claims as dependent on claim 1, wherein the electrically operated pump is a diaphragm pump.

6. The irrigation system of any one of the preceding claims, further comprising a power sensor for determining at least one of a current and a voltage supplied to said electrically operated pump, and wherein the controller is arranged to determine the inflation flow rate based on the current and/or voltage supplied to the electrically operated pump, based on input from the power sensor.

7. The irrigation system of any one of the preceding claims, further comprising a pressure sensor arranged to detect the pressure in the inflatable retention member.

8. The irrigation system of claim 7, wherein the controller is further arranged to prohibit operation of the electrically operated pump when a pressure measured by the pressure sensor is above a pressure threshold.

9. The irrigation system of claim 7 or 8, wherein the controller is arranged to continuously determine the inflation fluid volume based on the inflation flow rate in combination with the pressure in the inflatable retention member.

10. The irrigation system of any one of the claims 7-9, wherein the controller is arranged to determine the deflation fluid volume based on the deflation flow rate in combination with the pressure in the inflatable retention member.

11. The irrigation system of any one of the claims 7-10, wherein the pressure sensor is arranged directly in the inflatable retention member, or in a conduit being in direct communication with the inflatable retention member.

12. The irrigation system of any one of the preceding claims, further comprising a memory, storing a set of inflation levels correlated to volume levels, and
a user interface arranged to receive input of a selected inflation level selected form said set of inflation levels, wherein the controller is arranged to operate the electrically operated pump until one of the selected inflation level and the volume safety level, has been reached.

13. The system of any one of the preceding claims, wherein the catheter is a rectal catheter, and wherein the catheter system is a rectal, anal or transanal irrigation system, said system further comprising:

a reservoir for an irrigation liquid;
tubing providing fluid communication between said reservoir and said catheter;
and
an electrically operated pump for directly or indirectly pumping irrigation liquid from the reservoir to the probe through said tubing, wherein said electrically operated pump is either the same as the electrically operated pump for pumping a fluid for inflation of the inflatable retention member, or a second pump.

14. The system of claim 13, wherein the electrically operated pump is arranged for indirect pumping of irrigation liquid from the reservoir to the probe.

15. The irrigation system of any one of the preceding claims, further comprising a control unit with a housing, said housing enclosing said controller, said electrically operated pump and said electrically operated valve.

Fig. 1

Fig. 2

Fig. 3

Fig. 4

Fig. 5

Fig. 6

Fig. 7

Fig. 8

Fig. 9

Fig. 10

Europäisches
Patentamt

European
Patent Office

Office européen
des brevets

# EUROPEAN SEARCH REPORT

**Application Number**

EP 23 20 2446

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/143372 A1 (HVID NIELS [DK] ET AL) 12 May 2022 (2022-05-12) * paragraphs [0037] - [0069]; figures 1-5 * | 1-15 | INV. A61M3/02 A61M25/10 |
| A | US 2019/224402 A1 (HENRY JEROME A [IE] ET AL) 25 July 2019 (2019-07-25) * paragraphs [0098] - [0122]; figures 1-15 * | 1-15 | |
| A | US 10 569 009 B2 (DENTSPLY IH AB [SE]) 25 February 2020 (2020-02-25) * column 3, lines 30-53 * * column 11, line 51 - column 17, line 31; figures 1-5 * | 1-15 | |

**TECHNICAL FIELDS SEARCHED (IPC)**

A61M

The present search report has been drawn up for all claims

| Place of search | Date of completion of the search | Examiner |
|---|---|---|
| The Hague | 22 March 2024 | Schlaug, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 2446

22-03-2024

| Patent document cited in search report | | | Publication date | Patent family member(s) | | | Publication date |
|---|---|---|---|---|---|---|---|
| US 2022143372 | A1 | | 12-05-2022 | CN | 113677380 | A | 19-11-2021 |
| | | | | EP | 3952949 | A1 | 16-02-2022 |
| | | | | US | 2022143372 | A1 | 12-05-2022 |
| | | | | WO | 2020207547 | A1 | 15-10-2020 |
| US 2019224402 | A1 | | 25-07-2019 | AU | 2017292874 | A1 | 24-01-2019 |
| | | | | CA | 3030143 | A1 | 11-01-2018 |
| | | | | DK | 3481460 | T3 | 22-06-2020 |
| | | | | DK | 3593831 | T3 | 16-10-2023 |
| | | | | EP | 3481460 | A1 | 15-05-2019 |
| | | | | EP | 3593831 | A1 | 15-01-2020 |
| | | | | ES | 2808105 | T3 | 25-02-2021 |
| | | | | HU | E050845 | T2 | 28-01-2021 |
| | | | | JP | 6821779 | B2 | 27-01-2021 |
| | | | | JP | 2019520169 | A | 18-07-2019 |
| | | | | LT | 3481460 | T | 27-07-2020 |
| | | | | LT | 3593831 | T | 25-09-2023 |
| | | | | US | 2019224402 | A1 | 25-07-2019 |
| | | | | WO | 2018009871 | A1 | 11-01-2018 |
| US 10569009 | B2 | | 25-02-2020 | AU | 2017380905 | A1 | 06-06-2019 |
| | | | | BR | 112019012438 | A2 | 14-04-2020 |
| | | | | CA | 3045737 | A1 | 28-06-2018 |
| | | | | CN | 110087711 | A | 02-08-2019 |
| | | | | DK | 3338827 | T3 | 18-10-2021 |
| | | | | EP | 3338827 | A1 | 27-06-2018 |
| | | | | EP | 3928810 | A1 | 29-12-2021 |
| | | | | HU | E055716 | T2 | 28-12-2021 |
| | | | | JP | 6967592 | B2 | 17-11-2021 |
| | | | | JP | 2020503912 | A | 06-02-2020 |
| | | | | KR | 20190098160 | A | 21-08-2019 |
| | | | | US | 2018177937 | A1 | 28-06-2018 |
| | | | | WO | 2018114682 | A1 | 28-06-2018 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160193403 A
- EP 2683424 A
- US 20140052063 A
- WO 2016095929 A
- EP 3338827 A
- WO 2020142185 A
- US 9138516 B
- US 20060114148 A
- WO 2014154635 A
- US 20150335529 A